# EUROPÄISCHE PATENTSCHRIFT

(11) **EP 0 780 371 B1**
(45) Veröffentlichungstag und Bekanntmachung des Hinweises auf die Patenterteilung: **23.08.2000**
(21) Anmeldenummer: 96119753.0
(22) Anmeldetag: 10.12.1996
(51) Int. Cl.: C07C 323/62, C07C 319/14, C07C 67/03, C07C 69/76, C07C 51/15

(54) **2-Alkylmerkapto-4-(trifluormethyl)-benzoesäureester sowie ein Verfahren zu ihrer Herstellung**
2-Alkylmercapto-4-trifluoromethyl-benzoic acid esters as well as a process for their preparation
Esters d'acide 2-alkylmercapto-4-trifluorométhyl-benzoique ainsi qu'un procédé pour leur préparation

(30) Priorität: 22.12.1995 DE 19548428
(43) Veröffentlichungstag der Anmeldung: 25.06.1997
(73) Patentinhaber: Clariant GmbH, 65929 Frankfurt am Main (DE)
(72) Erfinder: Brungs, Peter, Dr., 65929 Frankfurt (DE); Karcher, Thomas, Dr., 84503 Altötting (DE); Kühlein, Klaus, Prof. Dr., 65779 Kelkheim (DE); Millauer, Hans, Dr., 65760 Eschborn (DE); Wildt, Manfred, 64753 Brombachtal (DE)

(56) Entgegenhaltungen:
- EP-A- 0 160 408
- EP-A- 0 524 018
- US-A- 3 855 285
- US-A- 3 867 407
- M. TROUPEL ET AL: "ELECTROORG. SYNTH. [Manuel M. Baizer Meml. Symp.] pages 355-360" 1991 , DEKKER , NEW YORK, N. Y., US XP000671378 * das ganze Dokument, besonders Seite 359, lines 1-14 *
- M. HEINTZ ET AL: TETRAHEDRON, Bd. 44, Nr. 6, 1988, Seiten 1631-1636, XP000673081

## Beschreibung

Die vorliegende Erfindung betrifft neue 2-Alkylmerkapto-4-(trifluormethyl)-benzoesäureester sowie ein Verfahren zu ihrer Herstellung.

2-Alkylmerkapto-4-(trifluormethyl)-benzoesäureester sind wertvolle Zwischenprodukte zur Herstellung eines Herbicides, welches in Maiskulturen eingesetzt wird (s. EP 527036, EP 470 856).

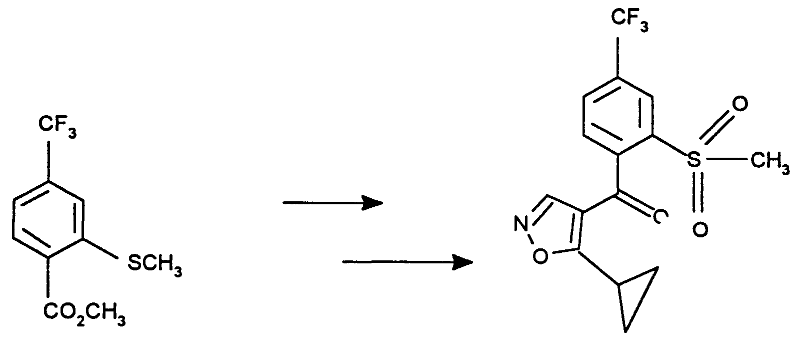

In der EP 524 018 (Reference Examples 17 und 18) und der EP 527 036 (Reference Example 6) ist die Herstellung von 2-Methylmerkapto-4-trifluormethylbenzoesäure auf folgenden Weg beschrieben.

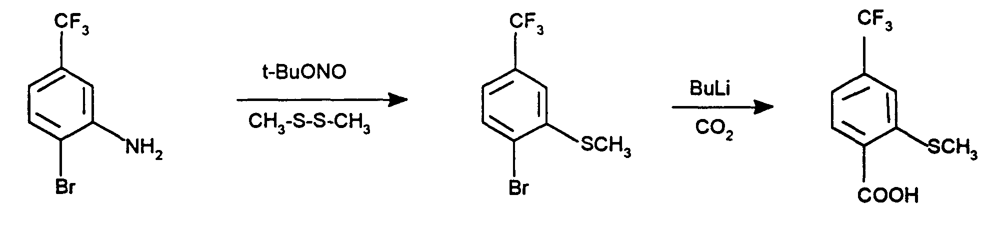

Nachteilig an dieser Methode ist das schwer zugängliche Ausgangsmaterial und die Verwendung von metallorganischen Reagenzien, die bei der Übertragung in größerem Maßstab zu sicherheitstechnischen Problemen führt.

Es bestand deshalb ein Bedarf nach einem Verfahren, das die beschriebenen Nachteile vermeidet, von gut zugänglichen Ausgangsmaterialien ausgeht und auch in großem Maßstab einfach durchzuführen ist.

Diese Aufgabe wird gelöst durch ein Verfahren zur Herstellung von 2-Alkylmerkapto-4-(trifluormethyl)-benzoesäureestern der Formel (II), worin R¹ und R² unabhängig voneinander (C₁-C₆) Alkyl bedeuten, dadurch gekennzeichnet, daß 3,4-Dichlorbenzotrifluorid elektrochemisch an einer Anode mit CO₂ zu 2-Chlor-4-(trifluormethyl)-benzoat umgesetzt, in den entsprechenden Benzoesäureester der Formel (I) überführt und anschließend mit einem Alkylmerkaptid R²S^{⊖} umgesetzt wird.

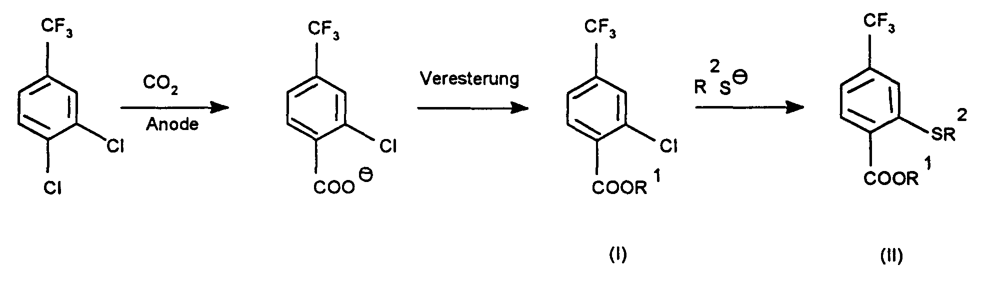

Der erste Schritt der Synthese von 2-Alkylmerkapto-4-(trifluormethyl)-benzoesäureestern beinhaltet die elektrochemische reduktive Carboxylierung von 3,4-Dichlorbenzotrifluorid zum Magnesium-2-chlor-4-(trifluormethyl)benzoat.

Man verwendet hierfür zweckmäßigerweise eine ungeteilte Elektrolysezelle, die von beliebiger Form sein kann, beispielsweise eine trogförmige Zelle oder eine Durchflußzelle, die mindestens eine Kathode und eine Anode aufweist.
Die Kathode besteht aus einem der üblichen Metalle, beispielsweise Aluminium, Magnesium, Eisen, Nickel, Chrom, Titan, Kupfer, Blei, Zink, Zinn, Cadmium, Silber, Gold oder Platin sowie Legierungen dieser Metalle oder aus Kohlenstoffmaterialien, beispielsweise Graphit oder glasartiger Kohlenstoff. Vorzugsweise finden Blei, Cadmium und Zinn Verwendung.
Als Anode werden Metalle eingesetzt, die unter den Elektrolysebedingungen schwer kathodisch abscheidbar sind, beispielsweise Aluminium, Calcium oder vorzugsweise Magnesium.

Als Elektrolyte kommen aprotische, dipolare Lösungsmittel in Betracht, beispielsweise Acetonitril, Dimethylacetamid, N-Methylpyrrolidon, Tetrahydrofuran oder vorzugsweise Dimethylformamid. Als Leitsalze finden indifferente, im Elektrolyten lösliche Salze, vorzugsweise Tetraalkylammoniumsalze Verwendung.
Während der Elektrolyse wird der Elektrolyt mit CO₂ gesättigt. Dies kann durch ständiges Durchleiten eines CO₂-Stromes oder durch Arbeiten in einer Druckzelle gewährleistet werden.

Die Elektrolyse wird bei Temperaturen zwischen etwa 0°C und 80°C, vorzugsweise zwischen 0°C und 20°C durchgeführt.
Die Elektrolyse erfolgt bei Stromdichten zwischen etwa 1 und 100 mA/cm², vorzugsweise zwischen 5 und 50 mA/cm².
Während der Elektrolyse wird zweckmäßig durch Rühren oder Strömen der Elektrolyt relativ zu den Elektroden bewegt.

Der zweite Schritt des Herstellverfahrens beinhaltet die Veresterung des elektrochemisch hergestellten 2-Chlor-4-(trifluormethyl)benzoats zu 2-Chlor-4-(trifluormethyl)benzoesäureestern.

Die Veresterung erfolgt vorzugsweise ohne vorangehende Aufreinigung des Rohelektrolyten in der Weise, daß man den eingeengten Rohelektrolyten mit dem zu veresternden Alkohol und einer starken Säure umsetzt und unter Rückfluß erhitzt.
Alternativ kann die Veresterung auch in einem 2-Phasensystem, bestehend aus Wasser und einem nicht mit Wasser mischbaren, organischen Lösungsmittel unter Verwendung starker Alkylierungsmittel, beispielsweise Dialkylsulfate, erfolgen.
Als Phasentransferkatalysatoren werden hierbei Tetralkylammoniumsalze, beispielsweise Tetrabutylammoniumhydrogensulfat, eingesetzt. Der pH-Wert wird durch Zugabe von Base bzw. durch Verwendung von Puffersystemen auf 7 gehalten.

Produkte der Formel (I) sind z.B. 2-Chlor-4-(trifluormethyl)-benzoesäuremethylester, 2-Chlor-4-(trifluormethyl)-benzoesäure-ethylester, 2-Chlor-4-(trifluormethyl)-benzoesäure-propylester, 2-Chlor-4-(trifluormethyl)-benzoesäureisopropylester, 2-Chlor-4-(trifluormethyl)-benzoesäure-1-butylester, 2-Chlor-4-(trifluormethyl)-benzoesäure-2-butylester, 2-Chlor-4-(trifluormethyl)-benzoesäure-isobutylester, 2-Chlor-4-(trifluormethyl)-benzoesäure-t-butylester, 2-Chlor-4-(trifluormethyl)-benzoesäure-amylester, 2-Chlor-4-(trifluormethyl)-benzoesäure-isoamylester.

Die erfindungsgemäße Darstellung der 2-Alkylmerkapto-4-(trifluormethyl)-benzoesäureester geschieht durch Umsetzung eines der oben aufgeführten 2-Chlor-4-(trifluormethyl)-benzoesäureesters mit einem Alkali-, Erdalkali- oder Ammonium-alkylmerkaptid in einem org. Lösungsmittel aus der Gruppe der Alkohole, Ether, Kohlenwasserstoffe, Aromaten oder einem anderen, dipolaren Lösungsmittel wie z.B. DMF.
Das Alkylmerkaptid kann auch in situ erzeugt werden, indem das freie Alkylmerkaptan in Gegenwart einer anorganischen oder organischen Base, wie z.B. Soda oder einem tert.-Amin, eingesetzt wird.

Die Umsetzung mit dem Alkylmerkaptid wird bei Temperaturen von 0°C bis 150°C durchgeführt. Die Isolierung der Verfahrensprodukte geschieht beispielsweise durch Verdampfen des Lösungsmittels und Isolierung des als Rückstand erhaltenen Rohproduktes durch fraktionierte Destillation oder Kristallisation.

Die Erfindung betrifft weiterhin Verbindungen der Formel (I), worin R¹ Ethyl bedeutet und Verbindungen der Formel (II) worin R¹ und R² (C₁-C₆) Alkyl bedeuten, insbesondere Verbindungen der Formel (II) mit R¹ gleich Ethyl und R² gleich Methyl.

Die nachfolgenden Beispiele dienen zur Erläuterung der Erfindung, ohne sie darauf zu beschränken.

### 1. Beispiel

Die reduktive Carboxylierung von 3,4-Dichlorbenzotrifluorid erfolgt elektrochemisch in einer ungeteilten Elektrolysezelle. Die Zelle besteht aus einem zylindrischen Glasgefäß mit Kühlmantel und einem eingeschliffenen Deckel aus Glas, in welchem sich 3 kleinere Schlifföffnungen befinden. Als Anode wird eine Magnesiumplatte (Länge: 110 mm; Breite: 55 mm; Eintauchtiefe: 100 mm) eingesetzt, als Kathode dient eine Cadmiumplatte mit den gleichen Abmessungen. Die Elektroden werden durch steife Drähte aus Platindraht, die als Stromzuführung dienen, im Deckel der Zelle gehalten. Die Zelle ist ferner mit einem Gaseinleitungsrohr für Kohlendioxid kombiniert mit einem Blasenzähler ausgestattet. Der Elektrolyt wird mit einem Magnetstab gerührt.

Die trockene Zelle wird mit 350 ml DMF, 21,5 g 3,4-Dichlorbenzotrifluorid und 2,7 g Tetrabutylammoniumbromid beschickt. Man leitet unter Rühren einen konstanten CO₂-Strom durch den Elektrolyten, um eine mit CO₂ gesättigte Lösung zu gewährleisten und startet nach einer 1/2 Stunde die Elektrolyse unter fortgesetztem Rühren und Durchleiten eines schwachen CO₂-Stroms. Die Stromstärke beträgt konstant 1,1 A, die Temperatur 15°C. Die Zellspannung liegt zunächst im Bereich von 3 bis 9 V und steigt gegen Ende auf ca. 25 V an. Die Ladungsmenge beträgt 2 A·s/V mol (2 F/mol).

Zur Veresterung der gebildeten Benzoesäure wird der Elektrolyt am Rotavapor bei 60°C/15 mbar bis zur Trockne eingeengt, der erhaltene Rückstand wird mit 250 ml Ethanol und 50 ml konz. H₂SO₄ versetzt und 8 h unter Rückfluß erhitzt. Die Reaktionslösung wird in 1,8 l Eiswasser gegeben und mit 200 ml Diethylether extrahiert. Die Etherphase wird getrocknet, filtriert und eingeengt. Nach Destillation des Rückstandes werden 11,9 g 2-Chlor-4-(trifluormethyl)-benzoesäureethylester erhalten.
- Sdp.:: 68 °C/6 - 10⁻⁴ bar
- H¹-NMR (300 MHz/CDCl₃): δ =: 1,42 (Triplett, J = 7Hz, CH₃)
4,44 (Quartett, J = 7Hz, CO₂CH₃)
7,56 (Dublett, J = 8Hz, 1Hₐᵣₒₘ)
7,71 (Singulett, 1Hₐᵣₒₘ)
7,91 (Dublett, J = 8Hz, 1Hₐᵣₒₘ) ppm

### 2. Beispiel

Analog Beispiel 1 wird aus dem Elektrolyten nach Einengung und Umsetzung mit n-Butanol / H₂SO₄ der 2-Chlor-4-(trifluormethyl)-benzoesäurebutylester erhalten.
- Sdp.:: 90°C 2 · 10⁻⁴ bar
H¹-NMR (300 MHz/CDCl₃): δ = 0,98 (Triplett, J = 6 Hz, CH₃); 1,49 (Quartett v. Tripletts, J = 6; 8 Hz, CH₂); 1,77 (Triplett v. Tripletts, J = 6; 8 Hz, CH₂); 4,39 (Triplett, J = 6 Hz; CO₂CH₂); 7,56 (Dublett, J = 8 Hz, 1Harom); 7,70 (Singulett, 1Hₐᵣₒₘ); 7,92 (Dublett, J = 8 Hz, 1Hₐᵣₒₘ) ppm.

### 3. Beispiel

Eine Lösung von 23,9 g (0,1 mol) 2-Chlor-4-(trifluormethyl)benzoesäuremethyl ester und 7,7 g (0,11 mol) Natrium-methanthiolat in 100 ml DMF wird 6 h bei 50°C gerührt. Zur Aufarbeitung wird das Reaktionsgemisch auf 400 ml Eiswasser gegeben und mit 150 ml Toluol extrahiert. Die org. Phase wird abgetrennt, über Na₂SO₄ getrocknet, filtriert und eingeengt. Nach Destillation des Rückstandes werden 18,7 g des 2-Methyl-merkapto-4-(trifluormethyl)-benzoesäuremethylesters erhalten.
- Sdp.:: 90°C / 2 · 10⁻⁴ bar
- Fp.:: 49 - 50°C
H¹-NMR (300 MHz/CDCl₃):
- d =: 2,50 (Singulett, SCH₃), 3,95 (Singulett, CO₂CH₃), 7,38 (Dublett, J = 8 Hz, 1 Hₐᵣₒₘ), 7,47 (Singulett, 1 Hₐᵣₒₘ), 8,08 (Dublett, J = 8 Hz, 1Hₐᵣₒₘ), ppm.
- MS:: m/e = 250

### 4. Beispiel

2,53 g (10 mMol) 2-Chlor-4-(trifluormethyl)benzoesäureethylester wird analog Beispiel 3 mit 0,77 g (11 mMol) Natrium-methanthiolat in 15 ml DMF umgesetzt. Man erhält 2,5 g 2-Methylmerkapto-4-(trifluormethyl)benzoesäureethylesters in hoher Reinheit. Auf eine destillative Aufreinigung kann daher verzichtet werden.
- Fp.:: 44 - 45°C
H¹-NMR (300 MHz/CDCl₃):
- d =: 1,42 (Triplett, CH₃), 2,50 (Singulett, SCH₃), 4,42 (Quartett, CO₂CH₂), 7,39 (Dublett, J = 8 Hz, 1Hₐᵣₒₘ), 7,48 (Singulett, 1 Hₐᵣₒₘ), 8,09 (Dublett, J = 8 Hz, 1Hₐᵣₒₘ) ppm.
- MS:: m/e = 264

### 5. Beispiel

Analog Beispiel 3 werden aus 12,7 g (5 mMol) 2-Chlor-4-(trifluormethyl)benzoesäureethylester 11,5 g 2-Propylmerkapto-4-(trifluormethyl)benzoesäureethylester dargestellt.
n²²_{D} = 1,5052
H¹-NMR (300 MHz/CDCl₃): δ = 1,10 (Triplett, J = 7 Hz, CH₃); 1,41 (Triplett, J = 7 Hz, CH₃); 1,78 (Quartett v. Tripletts, J = 7; 7 Hz, CH₂); 2,93 (Triplett, J = 7 Hz, SCH₂); 4,42 (Quartett, J = 7 Hz, CO₂CH₂); 7,34 (Dublett, J = 8 Hz, 1 Hₐᵣₒₘ); 7,52 (Singulett, 1 Hₐᵣₒₘ); 8,02 (Dublett, J = 8 Hz, 1Hₐᵣₒₘ) ppm.

### 6. Beispiel

Analog Beispiel 2 werden aus 12,6 g 2-Chlor-4-(trifluormethyl)benzoesäurebutylester (45 mMol) 11 g 2-Propylmerkapto-4-(trifluormethyl)benzoesäurebutylester dargestellt.
n²²_{D} = 1,4996
H¹-NMR (300 MHz/CDCl₃): δ = 0,98 (Triplett, J = 7 Hz, CH₃); 1,10 (Triplett, J = 7 Hz, CH₃); 1,49 (Quartett v. Tripletts, J = 8; 7 Hz, CH₂); 1,78 (Multiplett, 2 x CH₂); 2,94 (Triplett, J = 7 Hz, SCH₂); 4,36 (Triplett, J = 7 Hz, CO₂CH₂); 7,36 (Dublett, J = 8 Hz, 1 Hₐᵣₒₘ); 7,52 (Singulett, 1 Hₐᵣₒₘ); 8,03 (Dublett, J = 8 Hz, 1Hₐᵣₒₘ) ppm.

## Patentansprüche

1. Verfahren zur Herstellung von 2-Alkylmerkapto-4-(trifluormethyl)-benzoesäureestern der Formel (II), worin R¹ und R² unabhängig voneinander (C₁-C₆) Alkyl bedeuten, dadurch gekennzeichnet, daß 3,4-Dichlorbenzotrifluorid elektrochemisch an einer Anode mit CO₂ zu 2-Chlor-4-(trifluormethyl)-benzoat umgesetzt, in den entsprechenden Benzoesäureester der Formel (I) überführt und anschließend mit einem Alkylmerkaptid R²S^{⊖} umgesetzt wird.

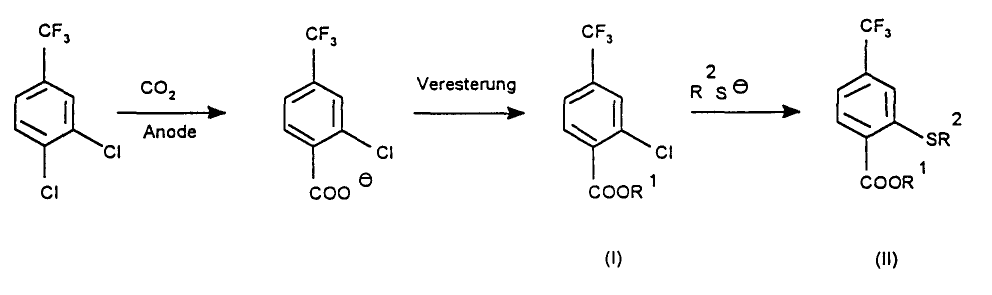

2. Verfahren nach Anspruch 1, dadurch gekennzeichnet, daß für die elektrochemische Umsetzung von 3,4-Dichlorbenzotrifluorid eine ungeteilte Elektrolysezelle verwendet wird, wobei die Kathode aus Aluminium, Magnesium, Eisen, Nickel, Chrom, Titan, Kupfer, Blei, Zink, Zinn, Cadmium, Silber, Gold, Platin, Legierungen dieser Metalle oder aus Kohlenstoffmaterialien und die Anode aus Aluminium, Calcium oder Magnesium besteht.

3. Verfahren nach Anspruch 2, dadurch gekennzeichnet, daß die Kathode aus Blei, Cadmium oder Zinn und die Anode aus Magnesium besteht.

4. Verfahren nach mindestens einem der Ansprüche 1 bis 3, dadurch gekennzeichnet, daß als Elektrolyt ein dipolar aprotisches Lösungsmittel, insbesondere Acetonitril, Dimethylacetamid, N-Methylpyrrolidon, Tetrahydrofuran oder Dimethylformamid, bevorzugt Dimethylformamid verwendet wird.

5. Verfahren nach mindestens einem der Ansprüche 1 bis 4, dadurch gekennzeichnet, daß der Elektrolyt während der Elektrolyse mit CO₂ gesättigt wird.

6. Verfahren nach mindestens einem der Ansprüche 1 bis 5, dadurch gekennzeichnet, daß die Elektrolyse bei Temperaturen von 0 bis 80°C, insbesondere 0 bis 20°C und Stromdichten von 1 bis 100 mA/cm², insbesondere 5 bis 50 mA/cm² durchgeführt wird.

7. Verfahren nach mindestens einem der Ansprüche 1 bis 6, dadurch gekennzeichnet, daß die Veresterung der 3-Chlor-4(trifluormethyl)-benzoesäure durchgeführt wird, indem man den eingeengten Rohelektrolyten mit dem Alkohol R¹OH und einer starken Säure, insbesondere HCI oder H₂SO₄ umsetzt.

8. Verfahren nach mindestens einem der Ansprüche 1 bis 6, dadurch gekennzeichnet, daß die Veresterung in einem 2-Phasensystem mit starken Alkylierungsmitteln, insbesondere Dialkylsulfaten unter Phasentransferkatalyse durchgeführt wird.

9. Verfahren nach mindestens einem der Ansprüche 1 bis 8, dadurch gekennzeichnet, daß als Alkylmerkaptid ein Alkali-, Erdalkali- oder Ammoniummerkaptid eingesetzt wird.

10. Verfahren nach mindestens einem der Ansprüche 1 bis 8, dadurch gekennzeichnet, daß das Alkylmerkaptid in situ aus Alkylmerkaptan und einer Base erzeugt wird.

11. Verfahren nach mindestens einem der Ansprüche 1 bis 10, dadurch gekennzeichnet, daß R² gleich Methyl ist.

12. Verfahren nach mindestens einem der Ansprüche 1 bis 11, dadurch gekennzeichnet, daß R¹ gleich Methyl oder Ethyl, insbesondere Ethyl ist.

13. Verbindungen der Formel (II) 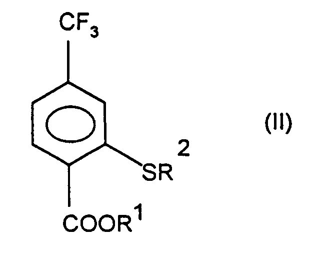 worin R¹ und R² (C₁-C₆)-Alkyl bedeuten.

14. Verbindung der Formel (II) gemäß Anspruch 13, worin R¹ gleich Ethyl und R² gleich Methyl ist.

15. Verbindungen der Formel (I) 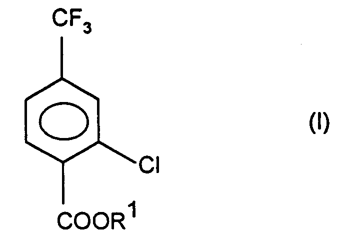 worin R¹ Ethyl bedeutet.

## Claims

1. A process for preparing 2-alkylmercapto-4-(trifluoromethyl)benzoic esters of the formula (II), where R¹ and R² are, independently of each other, (C₁-C₆) alkyl, which comprises reacting 3,4-dichlorobenzotrifluoride electrochemically at an anode with CO₂ to give 2-chloro-4-(trifluoromethyl)benzoate, converting it into the corresponding benzoic ester of the formula (I), and then reacting it with an alkylmercaptide R²S^{⊖} .

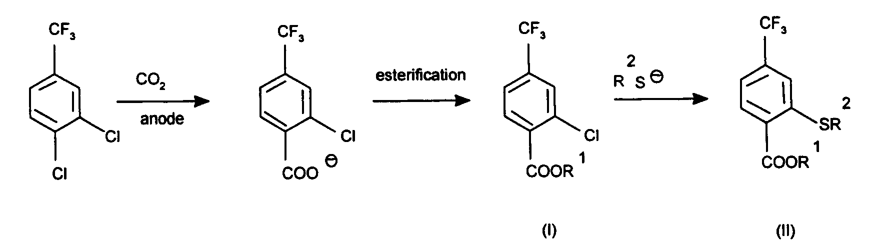

2. The process as claimed in claim 1, wherein the electrochemical conversion of 3,4-dichlorobenzotrifluoride is carried out in an undivided electrolytic cell where the cathode is made of aluminum, magnesium, iron, nickel, chromium, titanium, copper, lead, zinc, tin, cadmium, silver, gold, platinum, alloys of these metals, or carbon materials, and the anode of aluminum, calcium, or magnesium.

3. The process as claimed in claim 2, wherein the cathode is made of lead, cadmium, or tin, and the anode of magnesium.

4. The process as claimed in at least one of claims 1 to 3, wherein the electrolyte used is a dipolar aprotic solvent, in particular acetonitrile, dimethylacetamide, N-methylpyrrolidone, tetrahydrofuran or dimethylformamide, preferably dimethylformamide.

5. The process as claimed in at least one of claims 1 to 4, wherein the electrolyte is saturated with CO₂ during electrolysis.

6. The process as claimed in at least one of claims 1 to 5, wherein the electrolysis is carried out at temperatures of 0 to 80°C, in particular 0 to 20°C, and at current densities of 1 to 100 mA/cm², in particular 5 to 50 mA/cm² .

7. The process as claimed in at least one of claims 1 to 6, wherein the esterification of 3-chloro-4(trifluoromethyl)benzoic acid is carried out by reacting the concentrated crude electrolyte with the alcohol R¹OH and a strong acid, in particular HCl or H₂SO₄ .

8. The process as claimed in at least one of claims 1 to 6, wherein the esterification is carried out in a 2 phase system with strong alkylating agents, in particular dialkyl sulfates, under phase transfer catalysis.

9. The process as claimed in at least one of claims 1 to 8, wherein the alkylmercaptide used is an alkali metal mercaptide, an alkaline earth metal mercaptide or an ammonium mercaptide.

10. The process as claimed in at least one of claims 1 to 8, wherein the alkylmercaptide is formed in situ from alkyl mercaptan and a base.

11. The process as claimed in at least one of claims 1 to 10, wherein R² is methyl.

12. The process as claimed in at least one of claims 1 to 11, wherein R¹ is methyl or ethyl, in particular ethyl.

13. A compound of the formula (II) 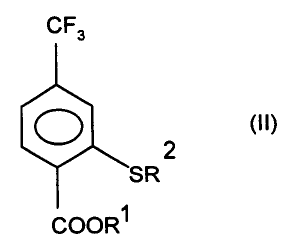 in which R¹ and R² are (C₁-C₆)-alkyl.

14. A compound of the formula (II)as claimed in claim 13 in which R¹ is ethyl and R² is methyl.

15. A compound of the formula (I) 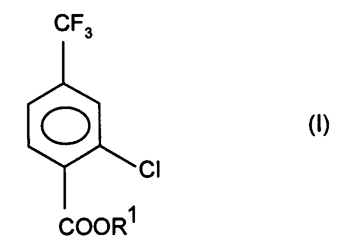 in which R¹ is ethyl.

## Revendications

1. Procédé de préparation de préparation d'esters d'acide 2-alkylmercapto-4-(trifluorométhyl)benzoïque de formule (II), où R¹ et R² représentent, indépendamment l'un de l'autre, des groupes alkyle en C₁-C₆, caractérisé en ce qu'on fait réagir le 3,4-dichlorobenzotrifluorure par voie électrochimique sur une anode avec du CO₂ pour obtenir le 2-chloro-4-(trifluorométhyl)-benzoate, on transforme en les esters de l'acide benzoïque correspondants de formule (I) et ensuite, on fait réagir avec un alkylmercaptide R²S^{Θ}.

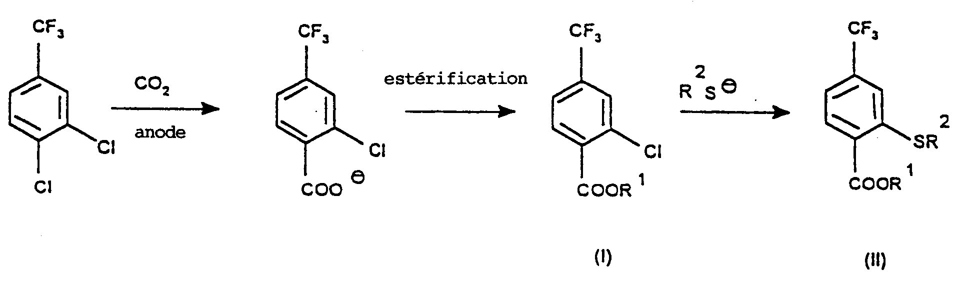

2. Procédé selon la revendication 1, caractérisé en ce qu'on utilise pour la transformation électrochimique du 3,4-dichlorobenzotrifluorure, une cellule électrochimique non divisée, la cathode étant en aluminium, magnésium, fer, nickel, chrome, titane, cuivre, plomb, zinc, étain, cadmium, argent, or, platine, alliages de ces métaux ou en matières carbonées et l'anode est en aluminium, calcium ou magnésium.

3. Procédé selon la revendication 2, caractérisé en ce que la cathode est en plomb, cadmium ou étain et l'anode est en magnésium.

4. Procédé selon au moins l'une des revendications 1 à 3, caractérisé en ce que l'électrolyte est un solvant aprotique dipolaire, en particulier l'acétonitrile, le diméthylacétamide, la N-méthylpyrrolidone, le tétrahydrofuranne ou le diméthylformamide, on utilise de préférence le diméthylformamide.

5. Procédé selon au moins l'une des revendications 1 à 4, caractérisé en ce que l'électrolyte est saturé par CO₂ au cours de l'électrolyse.

6. Procédé selon au moins l'une des revendications 1 à 5, caractérisé en ce qu'on met en oeuvre l'électrolyse à une température de 0 à 80°C, en particulier de 0 à 20°C et une densité de courant de 1 à 100 mA/cm², en particulier de 5 à 50 mA/cm².

7. Procédé selon au moins l'une des revendications 1 à 6, caractérisé en ce qu'on met en oeuvre l'estérification de l'acide 2-chloro-4-(trifluorométhyle)-benzoïque en faisant réagir l'électrolyte brut concentré avec l'alcool R¹OH et un acide fort, en particulier HCl ou H₂SO₄.

8. Procédé selon au moins l'une des revendications 1 à 6, caractérisé en ce qu'on met en oeuvre l'estérification dans un système biphasique avec des agents alkylants forts, en particulier des sulfates de dialkyles sous catalyse de transfert de phase.

9. Procédé selon au moins l'une des revendications 1 à 8, caractérisé en ce qu'on utilise en tant qu'alkylmercaptide un mercaptide alcalin, alcalinoterreux ou d'ammonium.

10. Procédé selon au moins une des revendications 1 à 8, caractérisé en ce qu'on produit l'alkylmercaptide in situ à partir d'alkylmercaptan et d'une base.

11. Procédé selon au moins l'une des revendications 1 à 10, caractérisé en ce que R² représente un groupe méthyle.

12. Procédé selon au moins l'une des revendications 1 à 11, caractérisé en ce que R¹ représente un groupe méthyle ou éthyle, en particulier éthyle.

13. Composé de formule (II) 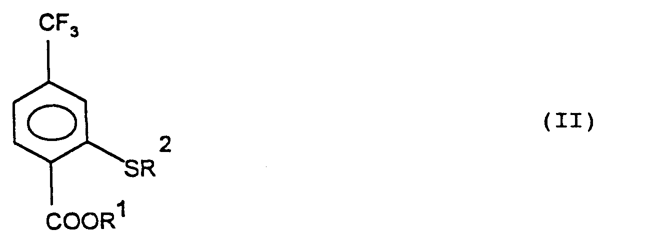 où R¹ et R² représentent un groupe alkyle en C₁-C₆.

14. Composés de formule (II) selon la revendication 13, où R¹ représente un groupe éthyle et R² représente un groupe méthyle.

15. Composés de formule (I) 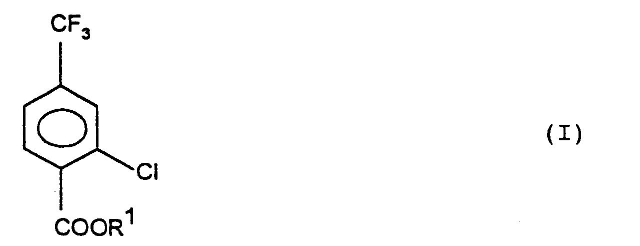 où R¹ représente un groupe éthyle.
